# EUROPEAN PATENT SPECIFICATION

(11) **EP 4 395 806 B1**
(45) Date of publication and mention of the grant of the patent: **22.10.2025**
(21) Application number: 22777702.6
(22) Date of filing: 27.08.2022
(51) Int. Cl.: A61K 38/16, A61P 1/02, A61P 1/04

(54) **PROTEIN COMPOSITIONS FOR THE TREATMENT OF ORAL MUCOSITIS**
PROTEINZUSAMMENSETZUNGEN ZUR BEHANDLUNG VON ORALER MUKOSITIS
COMPOSITIONS DE PROTÉINES POUR LE TRAITEMENT DE LA MUCOSITE ORALE

(30) Priority: 30.08.2021 IN 202121039213
(43) Date of publication of application: 10.07.2024
(73) Proprietor: Unichem Laboratories Ltd, Mumbai, Maharashtra 400102 (IN)
(72) Inventor: SATHE, Dhananjay, Thane Maharashtra 400601 (IN); MISHRA, Vivek, Sagar Madhya Pradesh 470001 (IN); JOG, Sunil, Mumbai Maharashtra 400057 (IN); BAKSHI, Gautam, Ambala city Haryana 134003 (IN)
(74) Representative: Marks & Clerk LLP
(86) International application number: PCT/IB2022/058039
(87) International publication number: WO 2023/031751

(56) References cited:
- EP-B1- 0 942 741
- WO-A1-2020/074984
- WO-A1-2020/104911
- WO-A1-99/11278
- WO-A2-2020/044296
- US-A1- 2016 228 498
- HUNTER A ET AL: "Treatment of oral mucositis after peripheral blood SCT with ATL-104 mouthwash: results from a randomized, double-blind, placebo-controlled trial", BONE MARROW TRANSPLANTATION, NATURE PUBLISHING GROUP, GB, vol. 43, no. 7, 10 November 2008 (2008-11-10), pages 563 - 569, XP037760909, ISSN: 0268-3369, [retrieved on 20081110], DOI: 10.1038/BMT.2008.363

## Description

### FIELD OF INVENTION

The present invention relates to pharmaceutical compositions of lectin proteins for use in the prevention, treatment and cure of oral mucositis caused by chemotherapy and/or radiotherapy.

### BACKGROUND OF INVENTION

Inflammation contributes in protecting injured tissue from further infection by triggering a multifactorial network of chemical signals which initiate the healing process. This involves the activation and migration of important blood cells (neutrophils, mast cells and eosinophils) to the site of damage. The cells (macrophages, mast cells, endothelial cells and Schwann cells) secrete cytokines and specific growth factors required for regeneration of cells and the process eventually results in reconstitution of the normal microenvironment. Cytokines and growth factors affect a wide variety of physiological processes such as cell proliferation, differentiation, apoptosis, immunological or hematopoietic response, morphogenesis, angiogenesis, metabolism, wound healing, and maintaining tissue homeostasis.

Methods of cancer treatment can include radiation therapy and chemotherapy, among others. Radiation therapy can cause inflammation of tissues and organs in and around the body site irradiated. Radiation can inflame skin to cause a burn or permanent pigmentation. Chemotherapy is known to damage cells in the mucous membrane.

Cells of the mucous membrane become inflamed (a condition called mucositis) due to disruption or loss of rapidly dividing epithelial progenitor cells lining the gastro-intestinal tract, leaving the mucosal tissue open to ulceration. This can lead to painful ulcers, bleeding and infection.

Severity of the inflammation varies depending upon a variety of factors, including the dose of medication, dose interval, the volume of treated tissue and the type of radiation.

Chemotherapy-induced enteritis, pancreatitis, peripheral neuropathy, phlebitis/ infusion phlebitis (Ph), and sarcopenia are common forms of inflammation due to chemotherapy.

Radiotherapy-induced radiation enteritis, vasculitis of iliac veins, optic neuropathy, proctitis, radiation pneumonitis, and skin reaction (RISR) are common forms of inflammation due to radiotherapy.

Inflammations due to chemotherapy and radiotherapy are severe inflammations that may lead to secondary cancers but are never addressed since the prime focus is on the treatment of cancer.

The most common type of inflammation as a side effect of chemotherapy and/or radiotherapy is oral mucositis (OM). It is a debilitating complication of cancer treatments, as it leads to pain, nutritional problems as a result of inability to eat, and increased risk of infection due to open sores in the mucosa.

Current clinical management of oral mucositis is mainly focused on palliative measures such as pain management, nutritional support, and hydration, use of saline rinses, topical and systemic pain relief and infection surveillance and treatment and maintenance of good oral hygiene.

Apart from this there are several promising therapeutic agents that are in various stages of clinical development for the management of oral mucositis.

Currently KepivanceTM (palifermin, US6677301) is a mucocutaneous epithelial human growth factor indicated to decrease the incidence and duration of severe OM in patients with hematologic malignancies receiving myelotoxic therapy in the setting of autologous hematopoietic stem cell support as an intravenous injection. However, there are several limitations towards use of KepivanceTM in the treatment or OM. Firstly, the safety and efficacy of KepivanceTM has not been established in patients with non-hematologic malignancies, and secondly it is not effective in decreasing the incidence of severe mucositis in patients with hematologic malignancies receiving myelotoxic therapy in the setting of allogeneic hematopoietic stem cell support. Besides, its high cost is a limitation in its affordability in the treatment or prevention of OM.

Other compounds have been evaluated for use as a prophylaxis and treatment of OM. Analgesics such as lidocaine mouthwashes are effective for short periods of time but within hours the pain and discomfort usually returns. Brilacidin (see international patent application WO2012158672), Mosedipimod (see WO2017082629) and Dusquetide (see WO2015038264) are some of the molecules which are under investigation for oral inflammation due to chemotherapy and/or radiotherapy.

Unfortunately, as of today there is not a single affordable pharmaceutical composition that is capable of preventing, reducing, or eliminating severe side effects such as inflammation caused by chemotherapy and/or radiotherapy in an efficient way. Patients with severe pain due to cancer and its treatment require an easy to use or apply, effective, cheap and highly efficient option for prevention and treatment of side effects of cancer treatments. With considerable increase in the cancer patient population recently, an effective treatment against side effects caused by cancer treatments especially inflammation due to chemotherapy and/or radiotherapy is an urgent need. The applicant's earlier patent application, 350/MUM/2009 (granted as patent IN 277986), discloses a recombinant lectin with the amino acid sequence of SEQ ID NO: 2 having high binding specificity toward the tumor-associated glycan TF antigen (Gal(31-3GalNAc-ct-O-Ser/Thr). In another application WO2020074984 (Al) the applicant disclosed use of lectin protein such as SEQ ID NO: 2 for the treatment and prevention of inflammatory diseases including psoriasis, psoriatic arthritis, rheumatoid arthritis (RA), hepatitis, asthma, inflammatory bowel disease, temporal arteritis, atopic dermatitis, systemic lupus, multiple sclerosis or sarcoidosis. Further, applicant in its application WO2021005500 (Al) discloses stable formulations of recombinant lectin protein such as SEQ ID NO: 2, which are preferably administered parenterally.

The formulations disclosed in WO2021005500 (Al) may have beneficial effects in the treatment of inflammations in a subject undergoing cancer treatment. However, the patient anguished due to chemotherapy or radiotherapy or both or any other such treatments would prefer a highly effective and easy to use formulation to treat side effects of such major treatments.

WO2020/104911 A1 describes a combination therapy using a recombinant lectin protein and another anticancer agent.

WO2020/044296 A2 describes a modified lectin protein.

WO99/11278 A1 describes *Robinia pseudoacacia* (RPA) lectin for use in medicine.

EP 0 942 741 B1 describes the use of lectins in the control of mucosal cell proliferation.

Hunter et al. 2008 (Bone Marrow Transplantation, Nature Publishing Group vol. 43, no. 7, pages 563-569) describes a study investigating the role of ATL-104 (the L-form of phytohaemagglutin) in the treatment of oral mucositis following treatment with chemotherapeutic agents.

WO2020/074984 A1 describes the use of a lectin in treating, reducing the progression of, and curing inflammation in a subject in need thereof.

### OBJECT OF INVENTION

An object of this invention is to provide an effective pharmaceutical composition for the prevention or treatment of oral inflammation due to cancer treatment. The invention is defined by the appended claims.

Another object of the present disclosure is to provide recombinant protein for the prevention or treatment of inflammation due to cancer treatment.

Yet another object of the present disclosure is to provide a highly specific, effective and cheap option for the prevention and treatment of side effects caused by cancer treatment.

Yet another object of the disclosure is to provide an economical and effective method of prevention and treatment of inflammation caused due to chemotherapy and/or radiotherapy.

Yet another object of the disclosure is to provide a method to cure or reduce the effect of inflammation caused due to chemotherapy and/or radiotherapy in a subject suffering from cancer and/or undergoing cancer treatment.

Yet another object of the disclosure is to provide an effective topical pharmaceutical composition for the prevention and treatment of inflammation caused by chemotherapy and/or radiotherapy.

An object of the invention is to provide an effective topical formulation for the treatment or prevention of oral mucositis (OM) caused by chemotherapy and/or radiotherapy.

Another object of the invention is to provide a method to cure or reduce or prevent or treat OM caused by chemotherapy and/or radiotherapy.

### SUMMARY OF INVENTION

According to a first aspect of the present invention, there is provided a topical composition comprising a recombinant lectin protein having the amino acid sequence of SEQ ID NO: 2 for use in the prophylactic and/or therapeutic treatment of oral mucositis caused by chemotherapy and/or radiotherapy in a subject undergoing cancer treatment.

"Cancer treatment" or "Cancer therapy" or "treatment of cancer" herein refers to the use of surgery, radiation, medication and/or other therapies to treat a cancer, cure a cancer, shrink a cancer or stop the progression and metastasis of a cancer. Cancer treatment using medication refers to treatment using chemical substances such as cytotoxic drugs including small molecules, for example 5-Fluorouracil (5-FU), that are used to free the subject from cancer or inhibit the progression of cancer cells. Such medications are called chemotherapeutic drugs/agents and such treatment of cancer is said to be chemotherapy. Cancer can also be treated independently or along with chemotherapy using radiation. The cancer cells are exposed to radiation such as light or hea radiation, X rays or gamma rays with high energy to either kill them or to inactivate their DNA, which inhibits progression of cancer cells. Such treatment is known as radiotherapy. There are other methods of cancer treatment such as targeted therapies, hormone therapy or immunotherapy to which the cancer patient may be subjected either independently or along with chemotherapy and/or radiotherapy.

In some embodiments, the cancer treatment may include methods that are used to treat, prevent, reduce or ameliorate cancer in a subject. According to a particular aspect, the cancer treatment includes chemotherapy and/or radiotherapy, wherein such therapies are used either independently or together. These therapies may also be used along with or before or after any other methods of cancer treatments.

As used herein, the term "inflammation" relates to any type of inflammatory immune response in a subject caused while undergoing cancer treatment and which is result of side effects of cancer treatment. The inflammation is said to be induced by or due to the cancer therapy or methods of cancer treatment. The inflammation may be associated with a chronic inflammatory disease, or it may be acute inflammation. According to one particular aspect of the present disclosure the inflammation is an inflammation of the digestive tract. According to a more particular aspect of the present description the inflammation is an inflammation of upper digestive tract including oral inflammation, and even more particularly, in the present invention, the inflammation is oral mucositis.

The term "induce" or "cause" herein means to produce. In the context of the present disclosure, a method of cancer therapy causes or induces inflammation wherein the cancer therapy is a potential trigger for initiation and or progression of inflammation or inflammatory disease in a subject undergoing cancer treatment.

It will be understood that the term "treatment" comprises substantially curing (i.e. eliminating) the inflammation or an underlying chronic inflammatory disease, or reducing (either permanently or temporarily) the symptoms associated with the inflammation. Such symptoms may include swelling, pain, itching, heat, redness (e.g. of the skin), loss of function (e.g. of joints or limbs), and difficulty breathing. In embodiments, the term may comprise preventing the onset of the inflammation, the disease, and/or the symptoms ("prophylactic treatment" in contrast to "therapeutic treatment").

It will be understood that the term "prevention" may comprise preventing the onset of the inflammation or preventing or slowing the progression of the inflammation or of an underlying chronic inflammatory disease. In the present invention, and with reference to the above description of the term "treatment", the inflammation or inflammatory disease is oral mucositis.

Topical pharmaceutical compositions are, in general, medications that are applied to a particular place on or in the body. Topical administration refers to application of the composition to body surfaces such as the skin or mucous membranes to treat various conditions. Solutions, creams, foams, gels, films, lotions, and ointments are some examples of topical formulations.

In the use according to the present invention, the topical composition is formulated for application to the oral mucosa. In embodiments the use comprises administering an effective amount of the lectin protein which is in the range of about 0.1 mg/kg to about 50 mg/kg body weight of the subject.

### DEFINITION

The terms "cancer", "tumor" and "tumour", are used interchangeably in the present application, as would be understood by the person skilled in the art. Cancers or tumours result from abnormal cell growth. They form when the normal cells grow out of control and crowd out. Formation of tumours often affects the normal functioning of the tissue, organ or organism.

Cancer can start any place in the body and can also spread to other parts of the body. The spread of cancer cells is referred to as metastasis. Thus the term "cancer" encompasses both primary and metastatic cancers. As used herein, the term "cancer" includes, but is not limited to, solid tumors and blood borne tumors.

The term "cancer" includes diseases of the skin, tissues, organs, bone, cartilage. Examples of cancers that may be relevant to the present invention include, but are not limited to, cancer of the bile duct, bladder, bone, brain, breast, cervix, colon, oesophagus, gastrointestine (including the ileum, colon, rectum and/or anus), head, kidney, liver, lung, nasopharynx, neck, ovary, pancreas, prostate, skin, stomach, testis, tongue, thyroid, urachus, vagina & uterus. The cancer may be benign or malignant, and in any stage of malignancy.

The cancer may be a cancer of the epithelial tissues, non-epithelial tissues, the cells that make up the skin or the tissue lining the organs, cells of the immune system, connective tissue, or cells of the spinal cord or brain. The cancer may be a carcinoma. According to some aspects of the invention, the cancer is adenocarcinoma. The adenocarcinoma may be oesophageal, pancreatic, prostate, cervical, breast, colon or colorectal, lung, bile duct, vaginal, urachus or stomach adenocarcinoma.

"Mucositis" herein refers to an inflammation and ulceration of the mucous membranes lining the digestive tract from the mouth to the anus.Mucositis is a mucosal damage often caused due to dose-limiting, toxicity of cancer therapy.

"Oral mucositis" (or "OM") herein refers to an inflammation of the mucous membrane in the upper digestive tract especially mouth. The subject is said to be suffering from OM when there is a tissue swelling, pain, ulceration , feeling of dryness, burning, soft whitish patches or pus in the digestive tract including mouth or on the tongue and increased risk of infection due to open sores in the mucosa caused by radiation and/or chemotherapy wherein the subject is undergoing cancer treatment.

The term "lectin" as used herein refers to a carbohydrate-binding protein. The term "protein" as used herein refers to a polymer of amino acid residues. The term "amino acid" as used herein refers to naturally occurring and synthetic amino acids, as well as amino acid analogues and amino acid mimetics that have a function that is similar to the naturally occurring amino acids. Naturally occurring amino acids are those encoded by the genetic code and include the proteinogenic amino acids. Naturally occurring amino acids also include those modified after translation in cells. Synthetic amino acids include non-canonical amino acids such as selenocysteine and pyrrolysine. Typically synthetic amino acids are not proteinogenic amino acids. It is understood that amino acids can be grouped according to different biochemical properties. Examples include: the polar amino acids, the non-polar amino acids, the acidic amino acids and the basic amino acids. According to one aspect , the amino acid used for the amino acid modification is at least one selected from the group consisting of, but not limited to: polar, non-polar, acidic, basic, selenocysteine, pyrrolysine and non-canonical. The terms "homology" or "homologous" as used herein refer to two or more referenced entities that share at least partial identity over a given region or portion. Areas, regions or domains of homology or identity refer to a portion of two or more referenced entities that share homology or are the same. Thus, where two sequences are identical over one or more sequence regions they share identity in these regions. Substantial homology refers to a molecule that is structurally or functionally conserved such that it has or is predicted to have at least partial structure or function of one or more of the structures or functions (e.g., a biological function or activity) of the reference molecule, or a relevant/corresponding region or portion of the reference molecule to which it shares homology. According to one aspect, the percentage "homology" between two sequences is determined using the BLASTP algorithm with default parameters (Altschul et al. Nucleic Acids Res. 1997 Sep 1; 25 (17):3389-402). In particular, the BLAST algorithm can be accessed on the internet using the URL: https://blast.ncbi.nlm.nih.gov/Blast.cgi. According to an alternative aspect, for global sequence alignments, percentage homology between two sequences is determined using the EMBOSS Needle algorithm using default parameters. In particular, the EMBOSS Needle algorithm can be accessed on the internet using the URL: https://www.ebi.ac.uk/Tools/psa/emboss_needle/. Unless otherwise indicated, where reference is made to a percentage homology, this is determined using the BLASTP algorithm.

Unless otherwise indicated, the term "homology" is used interchangeably with the term "sequence identity" in the present specification. The term "topical composition" refers to a pharmaceutical composition that comprises pharmaceutically acceptable ingredients, and a recombinant protein, and is intended for application to the surface of the skin or mucosa, in contrast to compositions that are taken orally or via intravenous route of administration.

A topical composition is generally intended to have its intended effect at the site of application and does not result in significant concentrations of drug in the bloodstream or other tissues. The term "excipients" as used herein are the inactive or usually inert substances that are added to the formulation which serve as a vehicle or medium for the active ingredient but do not affect the therapeutic effect of the active ingredient. It may be used to provide desired consistency, to improve stability, and/or to adjust osmolality of the composition. Pharmaceutically acceptable excipients are excipients that are safe, non-toxic, and are acceptable for human pharmaceutical use. Such excipients are accepted and approved for human use by the regulatory bodies such as Food and Drug Administration (FDA) in US or European Medical Agency (EMA) in Europe or corresponding agencies across the world.

The term "Assay" relates to quantitative estimation of drug substance present in the drug product by suitable analytical method known in the art. In the context of the present invention "assay" is the analysis for determination of quantitative estimation of the lectin protein of SEQ ID NO: 2 in a composition by High Performance Liquid chromatography (HPLC) method.

The term "Bioassay" relates to an analytical method to determine the concentration or potency of a substance by its effect on living animals or plants (in vivo), or on living cells or tissues (in vitro). In the context of the present invention "bioassay" is the analysis for determination of concentration or potency of lectin protein of SEQ ID NO: 2 in a composition of the present invention.

The term "Peptide Mapping" relates to an identity test for proteins, especially those obtained by rDNA technology. It involves the chemical or enzymatic treatment of a protein, resulting in the formation of peptide fragments, followed by separation and identification of the resultant fragments in a reproducible manner.

### BRIEF DESCRIPTION OF THE ACCOMPANYING SEQUENCES

SEQ ID NO: 1: represents the native *S*. *rolfsii* lectin amino acid sequence.
SEQ ID NO: 2: represents a variant of the S. *rolfsii* lectin amino acid sequence (reported as Rec-2 in WO 2010/095143).
SEQ ID NO: 3: represents a variant of the *S*. *rolfsii* lectin amino acid sequence (reported as Rec-3 in WO 2010/095143).
SEQ ID NO: 4: represents a variant of the *S*. *rolfsii* lectin amino acid sequence (reported in WO 2014/203261).
SEQ ID NO: 1 has the following sequence: 
TYKITVRVYQTNPNAFFHPVEKTVWKYANGGTWTITDDQHVLTMGGSGTSGTLRFHAD
NGESFTATFGVHNYKRWCDIVTNLAADETGMVINQQYYSQKNREEARERQLSNYEVK
NAKGRNFEIVYTEAEGNDLHANLIIG
SEQ ID NO: 2 has the following sequence: 
TYKITVRVYQTNPDAFFHPVEKTVWKYANG GTWTITD DQHVLTMGGS GTS GTLRFHAD
NGESFTATFGVHNYKRWCDIVTNLAADETGMVINQQYYSQKNREEARERQLSNYQVK
NAKGRNFQIVYTEAEGNDLHANLIIG
SEQ ID NO: 3 has the following sequence: 
VYKITVRVYQTNPDAFFHPVEKTVWKYANGGTWSITDDQHVLTMGGSGTSGTLRFHA
DNGESFTATFGVHNYKRWCDIVTNLAADETGMVINQQYYSQKNREEARERQLSNYQV
KNAKGRNFQIVYTEAEGNDLHANLIIG
SEQ ID NO: 4 has the following sequence:

VYKITVRVYQTNPDAFFHPVEKTVWKYADGGTWSITDDQHVLTMGGSGTSGTLRFHA
DNGESFTATFGVHDYKRWCDIVTDLAADETGMVINQEYYSEKDREEARERQNSNYEVK
DAKGRNFEIVYTEAEGNDLHADLIIG

Differences in the sequence of SEQ ID NOs: 2-4 compared with the sequence of SEQ ID NO: 1 have been underlined.

### DESCRIPTION OF DRAWINGS:

Figure 1: Percentage change in body weight *(g)* of the groups [Analyzed by Two-way ANOVA followed by Bonferroni posttests# p<0.05, ##p<0.01: G1 compared to G2]
Figure 2: Effect of SEQ ID NO 2 on TNF-α levels
Figure 3: Effect of SEQ ID NO 2 on Interluekin-1β levels

### DETAILED DESCRIPTION OF THE INVENTION

The present invention relates to a pharmaceutical composition comprising recombinant lectin protein for use in the prophylactic or therapeutic treatment of oral mucositis caused by chemotherapy and/or radiotherapy in a subject undergoing cancer treatment. In the present invention, the pharmaceutical composition is a topical composition.

In the present invention, the lectin is derived from the soil borne phytopathogenic fungus *Sclerotium rolfsii* (S*.* rolfsii) and comprises or consists of an amino acid sequence according to SEQ ID NO: 2, wherein SEQ ID NO: 2 is a recombinant sequence.

By "recombinant lectin", it is meant that the lectin gene is transferred into a host cell (such as bacteria or yeast) and the gene is then expressed to produce the lectin. Methods for preparing recombinant proteins will be well-known to those skilled in the art. For example, a recombinant DNA molecule, such as a plasmid or viral vector, comprising a nucleic acid sequence encoding the lectin may be provided. The nucleic acid sequence may be operatively linked to a promoter which is capable of controlling expression of the lectin in a suitable host cell. The recombinant DNA molecule may be inserted into a suitable host cell using methods known in the art, for example by transformation. Suitable host cells include prokaryotic cells (e.g. *E*. *coli)* and both lower eukaryotic cells (e.g. yeast cells) as well as higher eukaryotic cells. The host cell can then be cultured under appropriate conditions, whereby the recombinant lectin is expressed. The recombinant lectin can thus be obtained by isolation as an expression product from the host cell. Recombinant proteins can be purified by conventional techniques known in the art, typically conventional chromatographic methods.

In an aspect of the present invention, there is provided a topical composition comprising a recombinant lectin protein as described above for use in the prophylactic or therapeutic treatment of oral mucositis caused by chemotherapy and/or radiotherapy in a subject undergoing cancer treatment.

The terms 'formulation', 'composition', 'pharmaceutical formulation' and 'pharmaceutical composition' are used interchangeably and refer to preparations which are in such a form as to permit the biological activity of the active ingredients to be effective, and therefore may be administered to a subject for prophylactic or therapeutic use, wherein the subject is preferably human. 'Active ingredients' as used herein refers to the recombinant lectin or recombinant protein having desired biological or therapeutic activity to free the subject from the disease or symptoms of disease or reduce the severity or extent of the disease or symptoms of disease or slow or delay the progression of the disease or slow, delay or prevent onset of the disease or symptoms of the disease. According to an aspect, the composition may be formulated as liquid, semi-solid, or solid. According to a particular aspect, the composition for use according to the present invention may be formulated as a liquid such as a solution or suspension, cream, lotion, liniment, gel, oil, suspension, ointment, paste, emulgel, drops, rinses, aerosol foam, film or spray, or powder.

According to the use of the invention a pharmaceutical composition of lectin protein is administered topically. According to an aspect, the pharmaceutical composition for use according to the present invention which is a topical composition may further comprise pharmaceutically acceptable excipients. According to an aspect, the excipients may be selected from buffer, stabilizer, polymer, solubilizer, cryoprotectants, lyoprotectants and emollients, bulking agents, diluents emulsifiers, surfactants, rheology control agents, antimicrobials, antioxidants, humectants, silicones, preservatives, film formers, chelating agents. The selection of excipients for preparation of a composition for use according to the present invention is well within the knowledge and understanding of the skilled person. Further suitable excipients may be selected using the standard references such as Handbook of Pharmaceutical Excipients (Rowe RC, Sheskey P, Quinn M. Pharmaceutical Press; 2009) and The Theory and Practice of Industrial Pharmacy (Lachman, L., Lieberman, H. A., &Kanig, J. L. 1976). Non-limiting examples of excipients are buffer such as sodium phosphate, sodium monohydrogen phosphate dehydrate, sodium dihydrogen phosphate dehydrate, sodium citrate, sodium acetate, tris(hydroxymethyl)aminomethane (TRIS), TRIS-sodium chloride, phosphate buffer such as dibasic potassium phosphate, monobasic potassium phosphate or histidine; cryoprotectant or lyoprotectantsuch asdextran, glycine, mannitol, polyvinylpyrrolidone (PVP), sorbitol, and trehalose; an emollient such as lanolin, hydrocarbons such as paraffin, petrolatum and mineral oil, humectants such as carboxylic acid and lactic acid and oils such as olive, cottonseed, corn, almond, peanut and coconut oil; emulsifiers such as, soy and egg lecithin, mono- and diglycerides, polysorbates, carrageenan, guar gum and canola oil; antioxidants such as vitamins C and E, selenium, and carotenoids such as beta-carotene, lycopene, lutein, and zeaxanthin; chelating agents, such as EDTA; a stabilizer may be selected from a protein stabilizer known to the skilled person or from the list of stabilizers listed in applicant's previous application WO2021005500. In an example the stabilizer may be and amino acid or its salt, a polyol, a sugar, a carbohydrate or a surfactant, either individually or combination thereof. Preservatives may be selected from benzyl alcohol, m- cresol, methyl paraben, phenol or combination thereof; tonicity modifier such as sodium chloride, dextrose, potassium chloride, calcium chloride, sucrose or combination thereof; a chelating agent such as ethylene diamine tetra acetic acid; an antioxidant such as ascorbic acid, and/or a cryoprotectant such as mannitol, ethylene glycol, glycerol, sucrose, trehalose, and/or dextrose; humactants such as glycerin, propylene glycol. Polymers or viscosity enhancing agent in the composition may include polymers such as polyethylene glycols (PEGs), dextran, hydroxyl ethyl cellulose (HEC), Sodium carboxy methyl cellulose (Sodium CMC) or PEG-4000 or combination thereof; and a protein such as human serum albumin or gelatin or combinations thereof. An amino acid may be *selected* from glycine, alanine, serine, threonine, cysteine, valine, leucine, isoleucine, methionine, proline, phenylalanine, tyrosine, tryptophan, aspartic acid, glutamic acid, asparagine, glutamine, histidine, lysine, arginine or combinations thereof. The amino acid may be L-amino acid or D-amino acid, preferably L-amino acid. The amino acid may be used as such or as its salt. The salt may be an alkali salt or an alkaline earth metal salts, ammonium salts, organic amine salts such as triethylamine salt or triethanolamine salt, arginine salt such as basic amino acid salts or acid salts for example, hydrochloride, hydrobromide, sulfate, nitrate, phosphate, mineral acid salts citric acid salt, oxalate, tartrate or any other salt of amino acid as known to the skilled person. According to anaspect, the composition of the present invention is a topical formulation.

According to a particular aspect, the composition comprises pharmaceutically acceptable excipients suitable for the topical formulation. Such suitable excipients may be selected from emulsifiers, surfactants, emollients, rheology control/modifier agents, antimicrobials, antioxidants, silicones, solubilizers or film formers. According to an aspect, the excipient for the liquid topical formulation may be selected from vehicle or solvent, anti-foaming agents, emulsifiers, wetting agents, surfactants, solubilizers and preservatives. In another aspect, excipients may be selected from buffers, viscosity enhancers or polymers, stabilizers, humectants, and solvents.

According to some aspects, the composition for use according to the present invention may comprise pharmaceutically acceptable excipients such as glycerol and/or polyethylene glycol.

According to a particular aspect, a pharmaceutical composition of recombinant lectin protein for topical application may comprise a lyophilized powder of recombinant protein in propylene glycol and glycerin.

According to the present invention, there is provided a pharmaceutical composition for topical application comprising a recombinant lectin having an amino acid sequence of SEQ ID NO: 2 for use as described herein.

According to some aspects, the composition for use according to the present invention may comprise from about 0.01% to about 2.5% of lectin (which is a lectin of SEQ ID NO: 2), and pharmaceutically acceptable excipients comprising from about 0.005% to about 1.7% of TRIS, from about 0.007% to about 2.4% of sodium chloride (NaCl), from about 0.1 to about 4% of Hydroxy ethyl cellulose (HEC), from about 20% to about 40% of propylene glycol & from about 8% to about 40% of glycerin, wherein in the percentage is weight by weight (w/w).

According to another aspects, the composition for use according the present invention may comprise from about 0.01% to about 2.5% of lectin (which is a lectin of SEQ ID NO: 2), and pharmaceutically acceptable excipients comprising from about 0.005% to about 1.7% of TRIS, from about 0.007% to about 2.4% of sodium chloride (NaCl), from about 3% to about 10% of Sodium carboxy methyl cellulose (Sodium CMC), from about 0.01% to about 4.5% of poloxamer, from about 4% to about 60% of propylene glycol, from about 0.01% to about 4.5% of sucrose & from about 0.01% to about 0.2% of L-arginine hydrochloride, wherein in the percentage is weight by weight (w/w).

In yet another aspect, the pharmaceutical composition for use according to the present invention may comprise from about 0.01% to about 2.5% of lectin ( which is a lectin of SEQ ID NO: 2), and pharmaceutically acceptable excipients comprising from about 0.005% to about 1.7% of TRIS, from about 0.007% to about 2.4% of sodium chloride (NaC1), from about 0.1 to 4% of Hydroxy ethyl cellulose (HEC), from about 3% to about 10% of Sodium carboxy methyl cellulose (Sodium CMC), from about 4% to about 60% of propylene glycol & from about 8% to about 40% of glycerin, wherein in the percentage is weight by weight (w/w).

Exemplarily, the pharmaceutical composition for use according to the present invention may comprise from about 0.01% to about 2.5% of lectin (which is a lectin of SEQ ID NO: 2), from about 0.005% to about 1.7% of TRIS, from about 0.007% to about 2.4% of sodium chloride (NaCl), from about 0.002% to about 0.5% of polysorbate-80, from about 30% to about 40% of propylene glycol and from about 30% to about 40% Poloxamer 407, wherein in the percentage is weight by weight (w/w).

In yet another example, the composition for use according to the present invention may comprise from about 0.01% to about 2.5% of lectin (which is a lectin of SEQ ID NO: 2), from about 0.005% to about 1.7% of TRIS, from about 0.007% to about 2.4% of NaCl, from about 0.002% to about 0.5% of polysorbate-80 and necessary amount of glycerol.

The pharmaceutical composition for use according to the present invention can be prepared by the conventional methods or the processes known to the skilled person.

Alternatively, the composition may be prepared by combining lectin protein in buffer with stabilizers followed by addition of one or more optional excipients such as gelling agent, tonicity agent, preservatives, antioxidant, coloring agent and/or flavoring agent. The pH of the composition may be adjusted from about 7 to about 8.5 using acid such as hydrochloric acid or base such as sodium hydroxide. To adjust weight or dilution, water may be added to the composition.

The pharmaceutical composition for use according to the present invention can be prepared by conventional methods or the processes known to the skilled person.

According to an aspect, the recombinant lectin protein or a pharmaceutical composition as described herein is used in the prophylactic or therapeutic treatment of inflammation in a subject undergoing cancer treatment. The inflammation may be associated with a chronic inflammatory disease, or it may be acute inflammation.

In the present invention, the inflammation or the chronic inflammatory disease is OM induced by chemotherapy and/or radiotherapy in the subject undergoing cancer treatment.

The recombinant lectin protein or a pharmaceutical composition comprising the lectin for use according to the present invention may have an anti-inflammatory effect.

According to some aspects, the recombinant lectin protein or a pharmaceutical composition comprising recombinant lectin protein for use according to the present invention may have anti-inflammatory effect in the subject undergoing cancer treatment using chemotherapy or radiotherapy.

In some aspects, the recombinant lectin protein or pharmaceutical composition comprising the lectin for use according to the present invention is capable of preventing and protecting the subject from inflammation caused while undergoing cancer treatment, wherein the cancer treatment includes chemotherapy and/or radiotherapy.

The recombinant lectin protein for use according to the present invention which is a lectin having an amino sequence of SEQ ID NO: 2 protected cells from damages caused by chemotherapeutic agents such as 5-Fluorouracil (5-FU) and radiation therapy such as gamma radiation (3.5 kGy). In *in-vitro* studies with oral (buccal) mucosal cells (TR146) when exposed to 5-FU, the percentage cytotoxicity due to chemotherapy reduced to 28.9% at 10 µg/mL and 21.6% at 50 µg/mL concentration of SEQ ID NO 2. Similarly, TR146 when exposed to gamma radiation (3.5 kGy), the percentage cytotoxicity due to radiotherapy reduced to 20.6% at 1 µg/mL and 18% at 10 µg/mL concentration of SEQ ID NO 2.

In a particular aspect, the recombinant lectin protein for use according to the present invention has anti-apoptotic effect in chemotherapy and/or radiotherapy induced OM.

Apoptosis is a type of programmed cell death. In normal circumstances it enables an organism to eliminate unwanted and defective cells through an orderly process. However, in case of diseases such as inflammation there is uncontrolled apoptosis of the cells. This may be due to several reasons such as dropping of mitochondrial membrane potential (MMP) or increase in the reactive oxygen species in the cells. Lowering of apoptosis at the site of inflammation has positive effects on reduction of inflammation and increasing the healing process. The recombinant lectin protein for use according to the present invention, which is a lectin having SEQ ID NO 2 reduced apoptosis of cells that was caused by the inflammation resulting from chemotherapeutic agent (5-FU) or gamma radiation therapy (3.5 kGy). The oral inflammation such as OM in human oral (buccal) mucosa (TR146) cells was induced using 5-FU and gamma radiation and then treated with the recombinant lectin having SEQ ID NO 2. The inflamed cells due to 5-FU exhibited lowering of mitochondrial membrane potential (MMP) whereas the cells that were treated with SEQ ID NO 2 exhibited increase in MMP by about 67% at 0.01 µg/mL to about 110% at 50 µg/mL as compared to the amount of MMP in inflamed cells. Similarly, the radiated cell when treated with SEQ ID NO 2 exhibited increase in MMP by around 25% at 0.01 µg/mL and by around 37% at 5011g/mL.

Further SEQ ID NO 2 decreased apoptotic cell population by 81.7% at 0.01 µg/mL as compared to cells treated with chemotherapeutic agent and by 62.2% at 10 µg/mL as compared to cells treated with radiotherapy.

Inflammation causes considerable increase in the apoptotic sub(G0/G1) cells. The recombinant lectin protein for use according to the present invention, which has the amino acid sequence of SEQ ID NO 2, reduced apoptotic sub(G0/G1) cell population in chemotherapy and radiotherapy induced inflammation in oral (buccal) mucosa cells (TR146) by 78% at 10 µg/mL and by 59.5% at 0.1 µg/mL, respectively.

According to some aspects, the recombinant lectin protein is capable of inhibiting or reducing the secretion of inflammatory markers such as cytokine, matrix metalloproteinase (MMP), Nuclear Factorkappa-light-chain-enhancer of activated B cells (NE-KB), wherein inflammation is particularly caused due to methods of cancer treatments.

As is known in the art, cytokines are small proteins involved in cell signaling. Cytokines include chemokines, interferons, interleukins, lymphokines, vascular endothelial growth factor (VEGF), and tumour necrosis factors (TNF). As used herein, "Interleukin" (IL) includes IL-1, IL-2, IL-6, IL-8, IL-10, IL-12, IL-13, IL-15, IL-17, IL-18, IL-1β, IL-17A, IL-17F, IL-22, IL-26, IL-23, IL-20, and IL-15. NF-kB nuclear factor kB is a potent transcription factor which regulates the inflammatory cytokine production and inflammation, whenever the cells are exposed to inflammation or stress stimulators such as radiation or chemotherapeutic agents. Once NF-kB is activated, it simultaneously activates the cytokine, cytokine receptor and cyclooxygenase genes resulting in inflammation.

Metalloproteinase (MMP) is another such significant biomarker that damages the epithelial basal membrane by degrading collagen matrix. As a response to the damage by radiation and/or chemotherapy, fibronectin breakdown occurs which in turn stimulates macrophages leading to activation of MMP.

Vascular endothelial growth factors (VEGF) biomarker is one of the clinical biomarkers of OM capable of promoting neovascularization. Both chemotherapy and/or radiotherapy influences VEGF production and subsequent angiogenesis.

Similarly, levels of other biomarkers such as receptor for advanced glycation endproducts (RAGE) or chemokine (C-C motif) ligand (CCL) are increased quickly at the sites of inflammation. Also, enzymes such as cyclooxygenase-2 are mediators of inflammatory pathways that are responsible for the cause of pain and swelling of inflammation and other painful conditions. Hence, controlling the expression of the clinical biomarkers of inflammation such as OMis one of the classical ways of controlling pathogenesis of OM.

The ability of a recombinant lectin protein to inhibit or reduce the secretion of inflammatory cytokines may be determined using standard techniques, including the methods described herein.

For example, the level of an inflammatory cytokine secreted following inflammation in the presence of the recombinant lectin protein can be determined and compared to the level of the cytokine secreted following inflammation in the absence of the recombinant lectin protein. Levels of cytokines in samples such as cell culture supernatants (for *in vitro* studies) or serum samples (for *in vivo* studies) can be determined using standard techniques such as ELISA. The recombinant lectin protein for use according to the present invention, which has the amino acid sequence of SEQ ID NO: 2, demonstrated downregulation of TNF-α, M MP-3, CCL-2, MMP-8, RAGE and VEGF against radiation induced inflammation in human oral (buccal) mucosa cells (TR146). Similarly, SEQ ID NO: 2 demonstrated lowering of levels of cytokines (IL-I2/IL-23p40, IL-6, IL-1β, TNF-α), VEGF, MMP-3 and NF-kB in human oral (buccal) mucosa cells (TR146) having inflammation due to chemotherapy, 5-FU.

According to a particular aspect, the recombinant lectin protein for use according to the present invention is effective in controlling inflammation by regulating or inhibiting the secretion or activity of inflammatory markers. The downregulation of these biomarkers by a recombinant lectin according to SEQ ID NO: 2 elucidates the therapeutic control over the prognosis of OM, specifically induced by chemotherapy and/or radiotherapy. All these biomarkers play a significant role during the onset of the disease.

According to another aspect, the recombinant lectin protein or a pharmaceutical composition comprising recombinant lectin protein for use according to the present invention may have considerable therapeutic effect on chemotherapy and/or radiotherapy induced oral mucositis .

The therapeutic effect is an effect of the recombinant lectin protein or its topical composition on chemotherapy and/or radiotherapy induced oral mucositis in a subject undergoing cancer treatment can result in treatment or prevention of theoral mucositis. The therapeutic effect can be studied by analyzing the effect of recombinant lectin protein or its pharmaceutical composition on a subject suffering from oral mucositis on the tangible symptoms of disease such as redness, swelling, itching, pain or on the biomarkers or receptors that are either secreted or inhibited due to the disease condition. Such analysis is done by visual inspections or by analysis of the levels of biomarkers using the methods known to the skilled person or by the methods used as described herein.

According to some aspects, the recombinant lectin protein or topical compositions for use according to the present invention have the effect of lowering or reducing the tangible symptoms such as redness or swelling or decreasing the levels of the biomarkers that are increased in the subject suffering from OM which is caused by chemotherapy or radiotherapy.

In *in-vivo* studies described herein, subjects such as hamsters suffering from OM due to 5-FU and radiation exhibited a reduction in mucositis score, histology score and also reduction in the levels of cytokines such as IL-1β and TNF-α when treated with the composition of recombinant lectin protein of SEQ ID NO: 2 as compared to the untreated subjects.

According to the present invention, there is provided a topical composition comprising a recombinant lectin protein having the amino acid sequence of SEQ ID NO: 2 for use in the prophylactic and/or therapeutic treatment of oral mucositis (OM) caused by chemotherapy and/or radiotherapy in a subject undergoing cancer treatment.

The subject may develop symptoms of OM of mouth and digestive tract while undergoing the cancer treatment or after such treatment.

The prophylactic or therapeutic treatment may comprise administering a therapeutically effective amount of the recombinant lectin protein or a pharmaceutical composition (which is a topical composition) comprising recombinant lectin protein to the subject. According to some aspects, the lectin or its pharmaceutical composition (which is a topical composition) is administered at a dose of from 0.1 mg/kg to 1000 mg/kg, from 0.5 mg/kg to 100 mg/kg or from 1 mg/kg to 50 mg/kg. It will be within the capabilities of the skilled person to determine an amount of lectin to be administered according to the nature of the condition being treated and the subject.

The pharmaceutically acceptable formulation (which is a topical composition) may comprise therapeutically effective amount of lectin and excipients. Exemplary excipients include emulsifiers and surfactants, emollients, rheology control agents, antimicrobials, antioxidants, silicones, solubilizers and film formers. In the present invention terms "formulation" and "composition" are used interchangeably.

The subject may be a mammalian subject. According to some aspects, the subject is human. In particular the subject is a human subject suffering from and being treated for cancer and is suffering from or seeking prevention from oral mucositis caused as a side effect or adverse effect of cancer treatment.

The present invention relates to the prophylactic or therapeutic treatment of chemotherapy induced and/or radiation induced oral mucositis using a topical composition comprising a recombinant lectin protein, wherein the lectin is a lectin having the amino acid sequence of SEQ ID NO: 2. The invention also relates to a use of the topical composition comprising a lectin according to SEQ ID NO: 2 in preventing the onset of or the progression of chemotherapy induced and/or radiation induced oral mucositis in the subject. It further relates to to the use of a topical composition comprising a lectin according to SEQ ID NO: 2 in curing or reducing the effect of chemotherapy induced and/or radiation induced oral mucositis in the subject.

### EXAMPLES

The following examples are given to demonstrate the best mode of performance of the invention.

The examples do not limit the invention in any manner.

### Example 1: To assess the cytoprotective effect of a recombinant lectin having the amino acid sequence of SEQ ID NO: 2

Non-cytotoxic concentration of recombinant lectin having the amino acid sequence of SEQ ID NO: 2 was determined by treating oral (buccal) mucosa cells (TR146) with varying concentration of SEQ ID NO: 2 for 24 hrs and determining the cell viability using MTT assay. SEQ ID 2 demonstrated >70% cell viability at concentrations ranging from 0.01 µg/ml - 100 µg/ml, therefore this concentration was said to be non-cytotoxic. Similarly the positive controls (PC) Epigallocatechin gallate (EGCG) (PC-1) was found to be non-toxic at concentration range 0.01 µM - 1011M and Amifostine (PC-2) was found to be non-cytotoxic at concentration range 1 µg/ml - 25 µg/ml.

Further the cytoprotective effect of SEQ ID 2 (Test item or TI) was determined by treating oral buccal mucosal cells (TR146) non-cytotoxic concentrations of recombinant lectin having the amino acid sequence of SEQ ID NO: 2, wherein the cells were exposed to damages (5-FU 5mM) for 1.25 h for chemotherapy induced inflammation and exposed to gamma radiation (3.5 kGy) using Cobalt 60 for radiation induced inflammation. The cell viability was assessed by (MTT) assay and compared with positive control (Cells treated with Amifostine, EGCG and EGF and negative assay (untreated cells), wherein the control was the cells that underwent chemotherapy/radiotherapy.

The protective effect of the recombinant lectin on survival of TR146 cells against 5-FU induced damage was determined using following calculations:
The viability of cells was determined as: % Cell viability = (100 - % Cytotoxicity); where, the percentage cytotoxicity corresponding to each treatment was calculated as: %Cytotoxicity = [(R-X)/R]*100, wherein X = Absorbance of cells treated with 5-FU or radiation damage/TI + 5-FU, R = Absorbance of Control cells (Untreated) and Percentage protection was calculated as: ((Absorbance of TI + 5-FU or radiation) - (Absorbance of 5-FU or radiation alone)/(Absorbance of Untreated)-(Absorbance of 5-FU alone)}* 100

The test items (1 µg/ml - 100 µg/ml) demonstrated significant (p<0.01) cytoprotective potential against 5-FU induced cytotoxicity as reflected by increase in cell viability from 56.8% - 78.4% against 5-FU induced cell damage. The results are as given in Table 1.

**Table 1: Cytoprotective effect of SEQ ID NO. 2 in oral (buccal) mucosa cells (TR146) against 5-FU induced damage**

| Sample | | Concentration | %cytotoxicity | %viabilit y | %Protection |
|---|---|---|---|---|---|
| | | Untreated | 0 | 100 | |
| | | 5-FU (5mM) | 58.4 | 41.6 | 0 |
| 5-FU (5mM)+ | EGCG µM | 0.01 | 47 | 53 | 19.5 |
| | | 0.1 | 33.6 | 66.4 | 42.4 |
| | | 1 | 30.4 | 69.6 | 47.9 |
| | | 10 | 44.2 | 55.8 | 24.2 |
| | Amifostine µg/ml | 1 | 56 | 44 | 4.1 |
| | | 5 | 41.8 | 58.2 | 28.8 |
| | | 10 | 26.1 | 73.9 | 55.2 |
| | | 25 | 36.5 | 63.5 | 37.5 |
| | SEQ NO ID 2 µg/ml | 1 | 43.2 | 56.8 | 26 |
| | | 10 | 28.9 | 71.1 | 50.6 |
| | | 25 | 28.6 | 71.4 | 51 |
| | | 50 | 21.6 | 78.4 | 63 |
| | | 100 | 27.4 | 72.6 | 53 |

Test items (0.01 µg/ml - 25 µg/ml) also demonstrated significant (p<0.001) cytoprotective potential against radiation induced cytotoxicity as reflected by increase in cell viability from 60.5% - 82%. A maximum of 54.1% cytoprotection was observed. The results are as given in Table 2.

**Table 2: Cytoprotective effect of SEQ ID NO. 2 in oral (buccal) mucosa cells (TR146) against radiation induced damage.**

| Sample | | Concentr ation | % Cytotoxicity | % viability | % Protection |
|---|---|---|---|---|---|
| | Untreated | | 0 | 100 | |
| | Radiated control | 3.5 kGy | 39.2 | 62.8 | 0 |
| Radiation (3.5 kGy)+ | EGF (ng/ml) | 10 | 32.1 | 67.9 | 18 |
| | | 50 | 21.8 | 78.2 | 44.4 |
| | | 100 | 21 | 79 | 46.5 |
| | | 200 | 35.7 | 64.3 | 8.8 |
| | Amifostine µg/ml | 1 | 36.2 | 62.1 | 7.7 |
| | | 5 | 30.5 | 69.5 | 22.2 |
| | | 10 | 22.3 | 77.7 | 43.1 |
| | | 25 | 34.5 | 65.5 | 12 |
| | SEQ NO ID 2 µg/ml | 0.01 | 38.2 | 60.5 | 2.5 |
| | | 0.1 | 33.6 | 66.4 | 14.1 |
| | | 1 | 20.6 | 79.4 | 47.4 |
| | | 10 | 18 | 82 | 54.1 |
| | | 25 | 35.7 | 64.3 | 8.9 |

### Example 2: anti-apoptotic effect of recombinant lectin having the amino acid sequence of SEQ ID NO: tin chemotherapy & radiotherapy induced inflammation

Oral buccal mucosal cells (TR146) were incubated overnight in 5% CO₂ incubator at 37°C for 24 h. After 24 h, cells were treated with non-cytotoxic concentrations of recombinant lectin having the amino acid sequence of SEQ ID NO2 (Test Item or TI) for 24 h. After 24 h of treatment, cells were exposed to damage (5-FU 5mM) for 1.25 h for chemotherapy induced inflammation and exposed to gamma radiation (3.5 kGy) using Cobalt 60 for radiation induced inflammation. After the exposure, the anti-apoptotic effect of SEQ ID NO 2 was determined by restoration of mitochondrial membrane depolarization by JC-1 staining and reduction in apoptotic cell populations (Annexin positive and subG0/G1 cells in cell cycle analysis).

After incubation, the supernatants were discarded and 100µl of JC1-dye solution (prepared by diluting 1 mM DMSO stock into 10 µM in 1xPBS) was added to each well. The cells *were* then incubated with the dye in CO₂ incubator at 37°C for 15 min, after which the supernatant was removed, and the cells were washed twice with 1xPBS. 100 µl of 1xPBS was finally added to each well. Red fluorescence (excitation 550 nm, emission 600 nm) and green fluorescence (excitation 485 nm, emission 535 nm) were measured using Biotek Synergy HT plate reader. The mitochondrial membrane potential (Awm) was calculated as the ratio of intensity of red fluorescence to intensity of green fluorescence. The percentage increase/restoration in mitochondrial membrane potential (MMP) was calculated as: % Increase = [(12-X)/R] *100 Where, X *=*Δψm corresponding to TI + 5-FU treated cells or TI + radiation treated cells; R = Δψm corresponding to control cells (FU damaged or Radiation damaged alone)

It was seen that the recombinant lectin having the amino acid sequence of SEQ ID NO: 2 (0.01 µg/ml - 100 µg/ml) demonstrated significant (p<0.001) increase in MMP by 66.7% - 106.2% as compared to control (5-FU alone) in chemotherapy induced OM as given in the Table 3 below:

**Table 3: Anti-apoptotic effect of SEQ ID NO. 2 in oral (buccal) mucosa cells (TR146) via restoration of mitochondrial membrane potential against 5-FU induced damage**

| Sample | | Concentr ation | MMP | % Increase in MMP (with respect to control) |
|---|---|---|---|---|
| Untreated | | | 21 | |
| Radiated control | | | 8.5 | 0 |
| 5FU+ | Amifostine (PC) µg/ml | 1 | 14.3 | 68.7 |
| | | 5 | 14 | 64.9 |
| | | 10 | 15.7 | 85.3 |
| | | 25 | 16.1 | 90 |
| | | 50 | 17.4 | 105.2 |
| | SEQ ID NO 2 (µg/ml) | 0.01 | 14.1 | 66.7 |
| | | 0.1 | 14.2 | 67.5 |
| | | 1 | 15.4 | 81.5 |
| | | 10 | 15.2 | 79.6 |
| | | 25 | 17.3 | 104.8 |
| | | 50 | 17.8 | 110.2 |
| | | 100 | 17.5 | 106.2 |

The recombinant lectin having the amino acid sequence of SEQ ID NO: 2 (1 µg/ml) demonstrated significant (p<0.05) increase in MMP by 36.6% as compared to control (Radiation alone) in radiation induced inflammation as shown in the table 4 below:

**Table 4: Anti-apoptotic effect of SEQ ID NO. 2 in oral (buccal) mucosa cells (TR146) via restoration of MMP against radiation induced damage.**

| Sample | | Concent ration | MMP | % Increase in MMP (with respect to control) |
|---|---|---|---|---|
| Untreated | | | 14.21 | |
| Radiated control | | | 4.61 | 0 |
| (3.5 kGy) | | | | |
| Radiation (3.5 kGy)^{÷} | EGF (PC-1) ng/ml | 50 | 6.53 | 41.8 |
| | | 100 | 6.14 | 33.4 |
| | | 200 | 5.47 | 18.8 |
| | Amifostine (PC) µg/ml | 0.1 | 5.90 | 28.2 |
| | | 1 | 6.43 | 39.7 |
| | | 10 | 6.23 | 35.2 |
| | SEQ ID NO 2 µg/ml | 0.01 | 5.76 | 25 |
| | | 10 | 5.43 | 17.8 |
| | | 50 | 6.29 | 36.6 |
| | | 100 | 4.97 | 7.9 |

To determine the reduction in apoptotic cell populations using annexin-v staining cells were gently harvested into pre-labeled sterile centrifuge tubes and centrifuged at 300 xg for 5-7 min. Supernatant were discarded and the pellet was resuspended in 200 µl of fresh culture medium. 100 µl of cell suspension was transferred into pre-labeled sterile centrifuge tubes. 100 µl of Annexin-V reagent was added to each tube and incubated for 30 min at RT in dark. Cells stained for Annexin-V were then transferred into 96-well plates and acquired on flow cytometer (Guava technologies). Percentage of Annexin-V positive cells was calculated as follows: = a% Annexin positive cells in 5-FU or radiation alone] - [% Annexin positive cells in (TI + 5-FU) or (TI + radiation)]/% Annexin positive cells in 5-FU or radiation alone}*100

The recombinant lectin having the amino acid sequence of SEQ ID NO: 2 (0.01 µg/ml - 10 µg/ml) demonstrated significant (p<0.01) reduction in apoptotic cell population by 81.7% 51.5% as compared to control (5-FU alone), as given in the table 5 below:

**Table 5: Anti-apoptotic effect of SEQ ID NO. 2 in oral (buccal) mucosa cells (TR146) via decrease in Annexin positive cell population against 5-FU damage**

| Sample | | Concent ration | % Apoptotic cells (Annexin +ve) | % Decrease in Apoptotic cells (with respect to 5-FU) |
|---|---|---|---|---|
| | | | | |
| Untreated | | | 3 | |
| 5FU (10mM) | | | 16.4 | 0 |
| 5FU+ | Amifostine (PC) µg/m1 | 1 | 2.4 | 85.7 |
| | | 10 | 2.6 | 84.5 |
| | | 50 | 2.8 | 82.9 |
| | SEQ ID NO 2 pig/m1 | 0.01 | 3 | 81.7 |
| | | 1 | 3.1 | 81.1 |
| | | 10 | 8 | 51.5 |
| | | 50 | 19.3 | -17.4 |
| | | 100 | 26 | -58.5 |

The recombinant lectin having the amino acid sequence of SEQ ID NO: 2 at concentration 1 µg/ml & 10 µg/m1 demonstrated significant (p<0.001) decrease in apoptotic cell population by 56% & 62.6%, respectively as compared to the control, as given in the table 6 below:

**Table 6: Anti-apoptotic effect of SEQ ID NO. 2 in oral (buccal) mucosa cells (TR146) via decrease in Annexin positive cell population against Radiation damage**

| Sample | | Concent ration | % Apoptotic cells | % Decrease in Apoptotic cells (with respect to radiation control) |
|---|---|---|---|---|
| Untreated | | | 2.5 | |
| Radiated control (3.5 kGy) | | | 12.5 | 8.4 |
| Radiation (3.5 kGy)+ | EGF (PC-1) ng/ml | 50 | 13.7 | 0 |
| | | 100 | 8.8 | 35.9 |
| | | 200 | 13.9 | -1.5 |
| | Amifostine (PC-2) µg/m1 | 0.1 | 7.2 | 47.3 |
| | | 1 | 9.2 | 32.6 |
| | | 10 | 5.1 | 63 |
| | SEQ ID NO | 0.01 | 14.3 | -4.8 |
| | 2 µg/m1 | 0.1 | 14.7 | -7.3 |
| | | 1 | 6 | 56 |
| | | 10 | 5.1 | 62.2 |

To determine the effect of test item on cell cycle, oral buccal mucosal cells (TR146) were incubated overnight in 5% CO₂ incubator at 37°C for 24 h. After overnight incubation, cells were treated with the recombinant lectin having the amino acid sequence of SEQ ID NO: 2 (Test Items (TI)) at non-cytotoxic concentrations for 24 h. After 24 h of treatment, cells were exposed to chemotherapy (5-FU 10 mM) for 24 h or radiotherapy (Gamma radiation (3.5 kGy) using Cobalt 60) for 2 h.

After incubation, cells were harvested by trypsinization and processed for Cell cycle assay. Accordingly, cells were harvested into prelabeled centrifuge tubes and centrifuged at 450 g for 5 min at room temperature (RT, low brake). The supernatants were removed carefully and discarded. 1 ml of 1X phosphate buffer saline (PBS) was added to the pellet and resuspended gently to make homogenous suspension. Cells were centrifuged at 450 g for 5 min, RT (low brake). The supernatant was carefully removed leaving behind approx. 100111 of PBS. Cells were resuspended gently yet thoroughly in residual PBS. Ice-cold 70% ethanol (100 µl) was added dropwise into cells in each tube while vortexing at low speed (fixation step). Cells stored at 4°C for 24 h were centrifuged at 450 g for 5 min, RT (low brake). The supernatant was carefully removed without touching the pellet and discarded. 1 ml of 1X PBS was added into pellet and resuspended gently. Cells were incubated for 1 minute at RT. Cells were centrifuged at 450 g for 5 min, RT (low brake). The supernatant was removed carefully without touching the pellet, leaving behind approx. 20-50 µl of PBS. 200 µl of cell cycle reagent was added into each tube.

Cells were resuspended gently and mixed. Cells were incubated for 30 min at RT in the dark. The stained samples were transferred into 96-well plates and acquired on flow cytometer (Guava technologies). Percentage of cells in Sub (GO/G1) phase were determined based on the concept that cell cycle reagent contains PI stain, which stains DNA of cells in different phases of cell cycle; Sub (GO/G1), Gl, S, G2 and M. Cells in Sub (G0/G1) phase correspond to apoptotic cells.

Percent inhibition in apoptotic cells is determined by the formula: =[(% Sub(G0/G1) cells in 5-FU/ radiation alone)-(% Sub(G0/G1) cells in TI + 5-FU/radiation)/% Sub(G0/G1) cells in 5-FU/radiation alone]* 100

Inhibition of Sub (GO/G1) cell population by test items as compared to control (5-FU or radiation alone) is given in Table 7 and 8 below.

**Table 7: Anti-apoptotic effect of SEQ ID NO. 2 in oral (buccal) mucosa cells (TR146) via decrease in Sub(GO/G1) cell population against 5-FU induced damage**

| Sample | | Concentr ation | % Apoptotic cells (Sub GO/G1) | % Decrease in Apoptotic cells (with respect to control) |
|---|---|---|---|---|
| Untreated | | | 4.9 | |
| 5FU (10mM) | | | 30.8 | 0 |
| 5FU+ | Amifostine (PC) µg/ml | 1 | 13 | 58 |
| | | 10 | 11.4 | 63.2 |
| | | 25 | 17 | 45 |
| | | 50 | 15.1 | 50.9 |
| | SEQ ID NO 2 µg/m1 | 0.1 | 11.4 | 63.1 |
| | | 10 | 6.8 | 78 |
| | | 25 | 14.2 | 54.1 |
| | | 50 | 40.7 | -32.0 |

**Table 8: Anti-apoptotic effect of SEQ ID NO. 2 in oral (buccal) mucosa cells (TR146) via decrease in Sub(GO/G1) cell population against Radiation induced damage.**

| Sample | | Concentrat ions | % Apoptotic cells (SubGO/G1) | % Decrease in Apoptotic cells (with respect to control) |
|---|---|---|---|---|
| Untreated | | | 0.3 | |
| Radiated control | | (3.5 kGy) | 35.3 | 0 |
| | EGF (PC-1) ng/ml | 50 | 10.8 | 69.5 |
| | | 100 | 16.4 | 53.5 |
| Radiation (3.5 kGy)+ | | 200 | 30.5 | 13.7 |
| | Amifostine (PC) µg/ml | 0.1 | 19.4 | 45.1 |
| | | 1 | 21.8 | 38.2 |
| | | 10 | 12.9 | 63.5 |
| | SEQ ID NO 2 µg/ml | 0.01 | 15.2 | 56.9 |
| | | 0.1 | 14.3 | 59.5 |
| | | 1 | 22.3 | 36.9 |
| | | 10 | 35.7 | -1.3 |

### Example 3: To determine the mechanism of action of recombinant lectin having the amino acid sequence of SEQ ID NO: 2 by Multiplex analysis.

Oral buccal mucosal cells (TR146) cells were incubated overnight at 37°C in 5% CO₂ incubator. After 24 h, cells sera were starved for 4 h and were treated with recombinant lectin having the amino acid sequence of SEQ ID NO: 2 (Test Item or TI) at different concentrations ranging from 0.01 µg/ml- 1001.(g/ml for 24h. The cells were then stimulated with 5-FU (10 mM) for another 24 h for chemotherapy and with gamma radiation (3.5 kGy) using Cobalt 60 for radiotherapy. After 24 h, cell culture supernatants were collected from each well and stored at -20°C till multiplex analysis. For NF-kB estimations, cell lysates were prepared. Levels of biomarkers were estimated using Magpix^{®} multiplex machine. The cells treated with Amifostine (along with 5-FU or radiation) were considered as positive control (PC). Untreated cells (5-FU or radiation alone treated) were used as negative control (NC).

The percent modulation in each sample was determined as follows:
[{ Concentration of biomarkers (pg/ml) in Control cells (5-FU or radiation alone treated) 20 Concentration of biomarkers (pg/ml) in SEQ ID NO: 2 + 5-FU or radiation treated cells)}/Concentration of biomarkers (pg/ml) in Control cells (5-FU or radiation alone treated)] *100 The test items demonstrated downregulation of cytokines (IL-12/IL-23p40, IL-6, IL-1β, TNF-α), VEGF, MMP-3 and NF-kB against chemotherapy induced levels (Table 9 below):

**Table 9: Percentage Inhibition of inflammatory markers by SEQ ID NO. 2 expressed in Oral (buccal) mucosa cell line (TR146) due to chemotherapy.**

| Sample | | | TNF-α | IL-6 | MMP-3 | IL-1β | IL-12/IL-23p40 | VGEF | NF-kB |
|---|---|---|---|---|---|---|---|---|---|
| 5FU (10 mM) | | | 0 | 0 | 0 | 0 | 0 | 0 | 0 |
| 5-FU+ | Amifostine µg/m1 | 1 | 10 | 7.2 | 22.6 | 14.2 | 0 | 27.9 | 100 |
| | | 10 | 13.3 | 8.9 | 23.4 | 19.5 | -2.1 | 35.4 | 48.2 |
| | | 50 | 10 | 35.2 | 26.8 | 46.6 | 13.4 | 39.6 | 14.2 |
| | SEQ ID NO 2 µg/ml | 0.1 | 13.3 | 5.4 | 22 | 5.5 | 13.5 | 25 | 88.6 |
| | | 10 | 10 | 7.8 | 29.5 | 3.6 | 33.8 | 24.4 | 98.4 |
| | | 25 | 10 | 18.2 | 30.6 | 11 | 36.8 | 28 | 59.7 |
| | | 50 | 13.3 | 38.6 | 27.3 | 29.1 | 42.6 | 37.8 | 53 |
| | | 100 | 6.6 | 42.2 | 8.8 | 23.5 | 36.6 | 41.7 | 84.3 |

The test items demonstrated downregulation of TNF-α, MMP-3, CCL-2, MMP-8, RAGE and VEGF against radiation induced levels (Table 10 below):

**Table 10: Percentage Inhibition of inflammatory markers by SEQ ID NO. 2 expression in Oral (buccal) mucosa cell line (TR146) due to radiotherapy**

| Sample | | | TNF-α | IL-6 | MMP -3 | CCL-2 | MMP -8 | IL-10 | RAG E | VG EF | NF-kB |
|---|---|---|---|---|---|---|---|---|---|---|---|
| Radiated control (3.5 kGy) | | | 0 | 0 | 0 | 0 | 0 | 0 | 0 | 0 | 0 |
| Radiation (3.5kGy)+ | Amifostine µg/m1 | 0.1 | 49.3 | 14.2 | 51.9 | 23.1 | 3.6 | 10.7 | 45.1 | 10.3 | 15.8 |
| | | 10 | 44.3 | 1.3 | 47.4 | 0.1 | 13 | 12.1 | 40.3 | 7.2 | 17.7 |
| | SEQ ID NO 2 µg/ml | 0.01 | 43.2 | -0.2 | 42.8 | 0.1 | 1.8 | -17.3 | 38.8 | 0.9 | 11.8 |
| | | 0.1 | 45.2 | 3.1 | 51.9 | 23.1 | 13.9 | -15.1 | 42.6 | -8.1 | 9.9 |
| | | 1 | 39.6 | 3.8 | 47.4 | 23.1 | 11 | -11.5 | 47.6 | 8.4 | 9.9 |
| | | 10 | 47.1 | 7.7 | 47.4 | 23.1 | 8.5 | -44.8 | 53.5 | 23.3 | 4 |
| | | 25 | 41 | 5.2 | 28.8 | 0 | 7.6 | -97.1 | 39.1 | 29.7 | -4 |

### Example 4: To evaluate therapeutic effect of SEQ ID NO 2 on 5-fluorouracil induced OM in hamsters by measuring mucositis score, biomarkers estimation and histopathology evaluations of cheek pouch.

SEQ ID NO 2 (Test item) was formulated as given in table 11:

**Table 11: Formulation of SEO ID NO 2**

| **Test Items as lyophilized powder (mg)** | **Vehicle (50% propylene glycol and 50% glycerin)(mL)** | **Concentration of Test Item (mg/mL)** | **100µ1 formulation contains (mg/100µL)** |
|---|---|---|---|
| 4.9 | 1 | 2.5 | 0.25 |
| 9.8 | 1 | 5 | 0.5 |
| 19.5 | 1 | 10 | 0.1 |

In-vivo experiments conducted in healthy golden Syrian hamsters wherein animals were grouped as below:

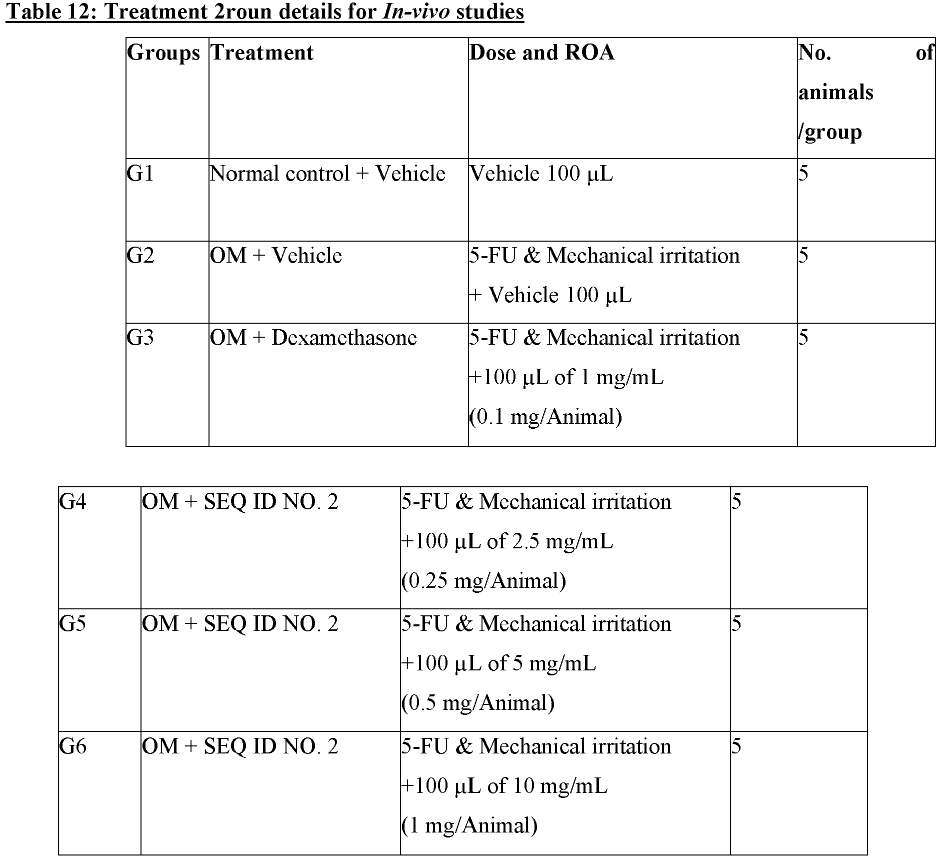

To induce OM, animals were administered (except normal control) 5-Fluorouracil (5-FU) by intraperitoneal route at doses of 60 mg/kg & 40 mg/kg respectively on first two days. On Day 4, under anesthesia, with aim of enhancing OM, mechanical irritation of the oral mucosa were done using wire brush. Treatment was initiated from Day 4 to Day 12. For group 3 animals 100 µ1 of *vehicle* comprising of standard drug Dexamethasone at 0.1 mg was instilled in buccal *cheek* pouch. For group 4, 5 and 6 animals 100 µ1 of vehicle comprising of SEQ ID NO. 2 at 0.25 mg, 0.5 mg & 1 mg, respectively was instilled in buccal cheek pouch. At the end of treatment period, cheek pouch of the hamsters was exposed, Mucositis scoring was carried out and images were captured. The animals were then humanely sacrificed, cheek pouch/ jugular mucosa were isolated and used for biomarker estimation and histopathological investigation by using ELISA method and Hematoxylin and Eosin (H&E) staining, respectively. Body weight of each animal was recorded daily till the end of experiment. Percentage change in body weight of the animals was calculated by using formula with respect to Day 4. % Body Weight Change = ((Mean body weight at day x - Initial Mean body weight)/ Initial Mean body weight} *100

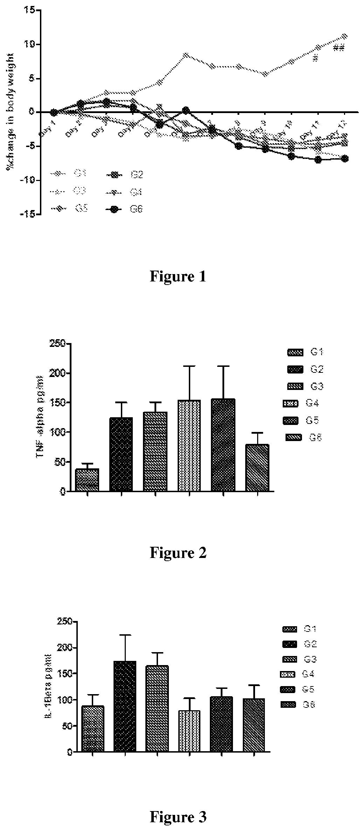

G1-Normal control group showed 11.19% gain in body weight. G2-OM group showed 4.46% decrease in body weight, which is significant on Day 11 (p<0.05), Day 12 (p<0.01) compared to group Gl-normal control group. Treatment groups (G2 to G6) showed non-significant decrease. G3 *- reference* standard dexamethasone 6.59%, G4 test item at 0.25/animal showed 3.61% decrease, G5 at 0.5/animal showed 4.36% & G6 at 1 mg/animal showed 6.8% decrease of body weight, compared to G2. Figure 1 depicts percentage change in body weight in all the groups.

Mucositis severity was scored as given below:
Cheek pouch completely healthy - 0; Mild erythema - 1;
Severe erythema and superficial erosion - 2; Formation of ulcers in one or more places - 3; Cumulative ulcer formation about 50% of cheek pouch surface area - 4; Complete ulceration of
the cheek pouch mucosa - 5.

The mucositis severity score for all the groups after 12 days treatment was: G1 - Normal control group showed completely healthy jugal mucosa;
G2 - OM group animals showed significant increase (p<0.001) in the score 4.00±0.5; G3 - dexamethasone group showed significant decrease (p<0.01) in the score 2.00±0.3;
G4 - Test item 0.25 mg/animal group showed Mucositis score 3.00±0.4;
G5 - Test items 0.5 mg/animal group showed Mucositis score 2.8±0.3; &
G6 - Test items 1 mg/animal group showed Mucositis score 4.00±0.3.

Further, for biomarker estimation jugular mucosa was dissected out, tissue weighed and stored in ice cold lx PBS. 100 mg of tissue was taken and in 1 ml of lx PBS buffer. Supernatant of homogenate was used for analysis of TNF-α and Interleukin-1β using ELISA method as per manufacturers instruction. The levels of TNF-α and Interleukin-1β are given in Table 13, Figure 2 and Table 14, Figure 3, respectively.

**Table 13: Effect of SEQ ID NO. 2 on TNF-α levels (pg/m1)**

| Groups | G1 | G2 | G3 | G4 | G5 | G6 |
|---|---|---|---|---|---|---|
| Mean | 56.90 | 124.00 | 134.29 | 153.48 | 155.76 | 79.37 |
| SE | 14.72 | 26.32 | 16.80 | 58.74 | 55.77 | 19.98 |
| % Increase vs G1 | NA | 117.92 | NA | NA | NA | NA |
| % Decrease vs G2 | NA | NA | -8.29 | -23.77 | -25.61 | 36.00 |

**Table 14: Effect of SEQ ID NO 2 on IL-10 levels**

| Groups | G1 | G2 | G3 | G4 | G5 | G6 |
|---|---|---|---|---|---|---|
| Mean | 87.44 | 173.67 | 163.96 | 79.10 | 105.66 | 101.96 |
| SE | 23.60 | 50.70 | 26.18 | 23.65 | 17.81 | 26.13 |
| % Increase vs G1 | NA | 98.62 | NA | NA | NA | NA |
| % Decrease vs G2 | NA | NA | 5.59 | 54.45 | 39.16 | 41.29 |

For histopathology the oral mucosa tissues were sectioned 5µm thickness and stained with hematoxylineosin staining. For study histopathological the sections were analyzed by light microscopy.

The pouch/jugular mucosa were observed and scored for infiltration of inflammatory cells, vasodilatation, presence of hemorrhagic areas, edema, ulcerations, and abscesses. The scoring system was as given below:

| **Score** | **Microscopic analysis of Oral mucosa** |
|---|---|
| **1** | Epithelium and connective tissue without vasodilation, absent or discrete cellular infiltration, and absence of bleeding, edema, ulcers, and abscesses |
| **2** | Discrete vasodilatation, areas of re-epithelialization, discrete cell infiltration with a high number of mononuclear leukocytes, and absence of hemorrhage, edema, ulcers, and abscesses; |
| **3** | Moderate vasodilatation, epithelial hydropic degeneration (vacuolization), moderate cellular infiltration with a predominance of polymorphonuclear leukocytes, presence of hemorrhagic areas, edema, and occasional small ulcers, and absence of abscesses; |
| **4** | Marked vasodilation, marked cell infiltration with a high number of polymorphonuclear leukocytes, presence of hemorrhagic areas, edema, and eventual ulceration, and absence of abscesses |
| **5** | Severe vasodilatation and inflammatory infiltration with neutrophils, abscesses, and extensive ulcer |

The Table 15 below shows Histopathology scores of the studied groups:

**Table 15: Histopathology score**

| **Groups** | **G1** | **G2** | **G3** | **G4** | **G5** | **G6** |
|---|---|---|---|---|---|---|
| Mean | 1 | 3.4# | 1.6 | 3 | 2 | 2.8 |
| SE | 0 | 0.4 | 0.5 | 0.6 | 0.4 | 0.5 |

| | | | | | | |
|---|---|---|---|---|---|---|
| Analyzed by One-Way ANOVA followed by Dunnett's Multiple Comparison Test #p<0.05 G2-OM group compared to G1-Normal control group. | | | | | | |

### Example 5: Topical Oral solution compositions

Topical oral solution compositions comprising lectin of SEQ ID NO: 2 were formulated with pharmaceutically acceptable excipients. Said compositions were prepared with pharmaceutically acceptable excipients comprising Buffer, Viscosity enhancer, humectants and solvent using below mentioned procedure.

Prepared compositions were evaluated for appearance and assay at 1 Month 25°C/60RH and at 3 Month 2°C-8°C stability conditions.

### Procedure:

Required quantity of water was taken and stirred using a magnetic stirrer.

Required quantity of viscosity enhancer was added slowly to the above container in vortex to obtain a clear solution.

Required quantity of humectant was added to the above solution with constant stirring.

Under stirring required quantity of lectin of SEQ ID NO: 2 was added to the above solution.

Above mixture was stirred continuously to obtain a clear solution. Volume or weight was made up with water.Mixture was stirred to obtain a uniform solution.

Below compositions were formulated using above manufacturing process

| **Table 16: Topical Oral solution compositionsof lectin of SEQ ID NO: 2.** | | | | | | | |
|---|---|---|---|---|---|---|---|
| Ingredient s | Cl (% w/v) | C2 (% w/v) | C3 (% w/v) | C4(% w/w) | C5 (% w/w ) | C6 (%w/w) | C7 (%w/w) |
| lectin of SEQ ID NO: 2 | 2.5 (0.25% ) | 2.5 (0.25% ) | 2.5 (0.25% ) | 2.5 (0.25% ) | 2.5 (0.25% ) | 2.5 (0.25%) | 2.5 (0.25%) |
| TRIS | 1.39 (0.13% ) | 1.39 (0.13% ) | 1.39 (0.13% ) | 1.41 (0.14% ) | 1.39 (0.13% ) | 1.39 (0.13%) | 1.39 (0.13%) |
| Sodium Chloride | 2.01 (0.20% ) | 2.01 (0.20% ) | 1.93 (0.19% ) | 2.04 (0.20% ) | 2.01 (0.20% ) | 2.01 (0.20%) | 2.01 (0.20%) |
| Hydroxyet hyl Cellulose | 20 (2%) | 20 (2%) | 20 (2%) | 20 (2%) | 20 (2%) | 20 (2%) | 20 (2%) |
| Glycerin | 100 (10%) | 126 (12.6% ) | 126 (12.6% ) | 126 (12.6% ) | 200 (20%) | - | 200 (20%) |
| Propylene Glycol | - | | | | | -300 (30%) | 300 (30%) |
| Water | q. s. up to 1 mL (q. s. up to 100%) | q. s. up to 1 mL (q. s. up to 100%) | q. s. up to 1 mL (q. s. up to 100%) | q. s. up to 1 g(q. s. up to 100%) | q. s. up to 1 g(q. s. up to 100%) | q. s. up q. to 1 g(q. s. up to 100%) | s. up to 1g(q. s. up to 100%) |

Observation: Prepared compositions were found to be of clear viscous solution appearance.

Prepared compositions when tested for assay at 1 Month 25°C/60RH and at 3 Month 2°C-8°C and were found to be in compliance with the regulatory standards (90-110%) for Assay. pH range of the prepared compositions were observed to be in the range from 7 to 9.

### Example 6: Topical Oral solution compositions

Topical oral solution compositions comprising lectin of SEQ ID NO: 2 were formulated with pharmaceutically acceptable excipients. Said compositions were prepared with pharmaceutically acceptable excipients comprising Buffer, Viscosity enhancer, stabilizers, humectants and solvent using below mentioned procedure. Prepared compositions were evaluated for appearance and assay at 1 Month 25°C/60RH and at 3

Month 2°C-8°C stability conditions. Procedure: In a beaker, required quantity of hot water was added to Sodium CMC and solution was stirred until clear, required quantity of propylene glycol was added and solution was stirred till clear solution was obtained.

To the above solution, required quantity of poloxamer 188 was added and stirred till clear solution was observed.

Above solution was thawed to room temperature. Required quantity of lectin of SEQ ID NO: 2 was added to the above solution and the solution was stirred until clear solution is observed. Volume or weight was made up with water. Mixture was stirred to obtain a uniform solution.

Below compositions were formulated using above manufacturing process

| **Table 17: Topical Oral solution compositions of lectin of SEQ ID NO: 2.** | | | |
|---|---|---|---|
| **Sr. No** | **Ingredients** | **C8 Quantity mg/ml (% w/v)** | **C9 Quantity mg/m1(% w/v)** |
| 1. | lectin of SEQ ID NO: 2 | 2.5 (0.25%) | 2.5 (0.25%) |
| 2. | TRIS | 1.39 (0.13%) | 1.39 (0.13%) |
| 3. | Sodium Chloride | 2.01 (0.20%) | 2.01 (0.20%) |
| 4. | Sodium CMC (Aqualon) | 50 (5 %) | 50 (5%) |
| 5. | Poloxamer 188 | 25 (2.5%) | 25 (2.5%) |
| 6. | Propylene glycol | 50 (5%) | 50 (5%) |
| 7. | Sucrose | 25 (2.5%) | |
| 8. | L-Arginine Hydrochloride | 1 (0.1%) | |
| 9. | Water | q. s. up to 1 mL (q. s. up to 100%) | q. s. up to 1 mL (q. s. up to 100%) |

**Observation:** Prepared compositions were found to be of clear viscous solution appearance. Prepared compositions when tested for assay at 1 Month 25°C/60RH and at 3 Month 2°C-8°C and were found to be in compliance with the regulatory standards (90-110%) for Assay. pH range of the prepared compositions were observed to be in the range from 7 to 9.

### Example 7: Topical Oral gel compositions

Topical oral gel compositions comprising lectin of SEQ ID NO: 2 were formulated with pharmaceutically acceptable excipients. Said compositions were prepared with pharmaceutically acceptable excipients comprising Buffer, Viscosity enhancing agent, stabilizers, humectants and solvent using below mentioned procedure.

### Procedure:

In a beaker, required quantity of water was taken and stirred using a mechanical stirrer. Required quantity of Propylene glycol and glycerin were added to the above container and was stirred for 10 minutes.

In a polybag, required quantity of Sodium CMC and Hydroxy ethyl cellulose was mixed and the mixture was slowly added to solution of water, propylene glycol and glycerin.

Resulting mixture was stirred until ingredients were dissolved properly, a thick gel appeared. A clear uniform gel was formed. Batch size was made up with water and mixture was stirred to get a uniform gel.

| **Table 18:Topical Oral gel compositions comprising lectin of SEQ ID NO: 2** | | |
|---|---|---|
| Sr. No. | Ingredients | C10 Quantity mg/g (%w/w) |
| 1 | lectin of SEQ ID NO: 2 | 2.5(0.25%) |
| 2 | TRIS | 1.39(0.13%) |
| 3 | Sodium chloride | 2.01(0.20%) |
| 4 | Hydroxy ethyl cellulose | 20(2%) |
| 5 | Sodium carboxymethyl cellulose | 20(2%) |
| 6 | Propylene glycol | 50(5%) |
| 7 | Glycerin | 300(30%) |
| 8 | Water | q. s. up to 1 gram (q. s. up to 100%) |

Observation: Prepared compositions were found to be of clear viscous solution appearance. pH range of the prepared compositions were observed to be in the range from 7 to 9.

## Claims

1. A topical composition comprising a recombinant lectin protein having the amino acid sequence of SEQ ID NO: 2 for use in the prophylactic and/or therapeutic treatment of oral mucositis caused by chemotherapy and/or radiotherapy in a subject undergoing cancer treatment.

2. The composition for use of claim 1 wherein the composition is formulated for topical application to the oral mucosa.

3. The composition for use of claim 1, wherein the composition comprises one or more pharmaceutically acceptable excipients and/or diluents selected from buffer, viscosity enhancer, stabilizer, and humectant.

4. The composition for use of claim 3, wherein the composition comprises ;
a. one or more buffers selected from TRIS buffer and sodium chloride;
b. one or more viscosity enhancing agents selected from Hydroxyethyl cellulose, Poloxamer-188 and sodium carboxy methyl cellulose;
c. one or more humectant selected from glycerin, propylene glycol;
d. one or more of stabilizers selected from sucrose, L-Arginine hydrochloride

5. The composition for use of claim 1, wherein the composition comprises of 0.01% to 2.5% lectin protein; 0.005% to 1.7% of TRIS; 0.007% to 2.4% of sodium chloride (NaCl); 0.1 to 4% of Hydroxy ethyl cellulose (HEC); 20% to 40% of propylene glycol; & 8% to 40% of glycerin.

6. The composition for use of claim 1, wherein the composition comprises 0.01% to 2.5% of lectin protein, 0.005% to 1.7% of TRIS, 0.007% to 2.4% of sodium chloride (NaCl), 3% to 10% of Sodium carboxy methyl cellulose (Sodium CMC), 0.01% to 4.5% of poloxamer, 4% to 60% of propylene glycol, 0.01% to 4.5% of sucrose; & 0.01% to 0.2% of L-arginine hydrochloride.

7. The composition for use of claim 1, wherein the composition comprises 0.01% to 2.5% of lectin protein, 0.005% to 1.7% of TRIS; 0.007% to 2.4% of sodium chloride (NaCl); 0.1 to 4% of Hydroxy ethyl cellulose (HEC); 3% to 10% of Sodium carboxy methyl cellulose (Sodium CMC); 4% to 60% of propylene glycol; & 8% to 40% of glycerin.

## Patentansprüche

1. Topische Zusammensetzung, die ein rekombinantes Lektinprotein umfasst, das die Aminosäuresequenz aus SEQ.-ID Nr. 2 aufweist, zur Verwendung in der prophylaktischen und/oder therapeutischen Behandlung von oraler Mukositis, die durch Chemotherapie und/oder Strahlentherapie verursacht wird, bei einem Patienten, der sich einer Krebsbehandlung unterzieht.

2. Zusammensetzung zur Verwendung nach Anspruch 1, wobei die Zusammensetzung für topische Anwendung auf der Mundschleimhaut formuliert ist.

3. Zusammensetzung zur Verwendung nach Anspruch 1, wobei die Zusammensetzung ein(en) oder mehrere pharmazeutisch verträgliche Hilfsstoffe und/oder Verdünnungsmittel umfasst, ausgewählt aus Puffer, Viskositätsverstärker, Stabilisator und Feuchthaltemittel.

4. Zusammensetzung zur Verwendung nach Anspruch 3, wobei die Zusammensetzung umfasst:
a. einen oder mehrere Puffer, ausgewählt aus TRIS-Puffer und Natriumchlorid;
b. ein oder mehrere Viskositätsverstärkungsmittel, ausgewählt aus Hydroxyethylcellulose, Poloxamer-188 und Natriumcarboxymethylcellulose;
c. ein oder mehrere Feuchthaltemittel, ausgewählt aus Glycerin, Propylenglykol;
d. einen oder mehrere Stabilisatoren, ausgewählt aus Saccharose, L-Argininhydrochlorid.

5. Zusammensetzung zur Verwendung nach Anspruch 1, wobei die Zusammensetzung 0,01 % bis 2,5 % Lektinprotein; 0,005 % bis 1,7 % TRIS; 0,007 % bis 2,4 % Natriumchlorid (NaCl); 0,1 bis 4 % Hydroxyethylcellulose (HEC); 20 % bis 40 % Propylenglykol; und 8 % bis 40 % Glycerin umfasst.

6. Zusammensetzung zur Verwendung nach Anspruch 1, wobei die Zusammensetzung 0,01 % bis 2,5 % Lektinprotein, 0,005 % bis 1,7 % TRIS, 0,007 % bis 2,4 % Natriumchlorid (NaCl), 3 % bis 10 % Natriumcarboxymethylcellulose (Natrium-CMC), 0,01 % bis 4,5 % Poloxamer, 4 % bis 60 % Propylenglykol, 0,01 % bis 4,5 % Saccharose; und 0,01 % bis 0,2 % L-Argininhydrochlorid umfasst.

7. Zusammensetzung zur Verwendung nach Anspruch 1, wobei die Zusammensetzung 0,01 % bis 2,5 % Lektinprotein, 0,005 % bis 1,7 % TRIS; 0,007 % bis 2,4 % Natriumchlorid (NaCl); 0,1 bis 4 % Hydroxyethylcellulose (HEC); 3 % bis 10 % Natriumcarboxymethylcellulose (Natrium-CMC); 4 % bis 60 % Propylenglykol; und 8 % bis 40 % Glycerin umfasst.

## Revendications

1. Composition topique comprenant une protéine lectine recombinante présentant la séquence d'acides aminés de SEQ ID NO : 2 pour une utilisation dans le traitement prophylactique et/ou thérapeutique de la mucosite buccale causée par une chimiothérapie et/ou une radiothérapie chez un sujet soumis à un traitement contre le cancer.

2. Composition pour une utilisation selon la revendication 1, dans laquelle la composition est formulée pour une application topique sur la muqueuse buccale.

3. Composition pour une utilisation selon la revendication 1, dans laquelle la composition comprend un ou plusieurs excipients et/ou diluants pharmaceutiquement acceptables sélectionnés parmi un tampon, un promoteur de viscosité, un stabilisant et un humectant.

4. Composition pour une utilisation selon la revendication 3, dans laquelle la composition comprend :
a. un ou plusieurs tampons choisis parmi le tampon TRIS et le chlorure de sodium ;
b. un ou plusieurs agents améliorant la viscosité choisis parmi l'hydroxyéthylcellulose, le poloxamère-188 et la carboxyméthylcellulose sodique ;
c. un ou plusieurs humectants choisis parmi la glycérine, le propylène glycol ;
d. un ou plusieurs stabilisants choisis parmi le saccharose et le chlorhydrate de L-arginine

5. Composition pour une utilisation selon la revendication 1, dans laquelle la composition comprend de 0,01 % à 2,5 % de protéine lectine ; de 0,005 % à 1,7 % de TRIS ; de 0,007 % à 2,4 % de chlorure de sodium (NaCl) ; de 0,1 à 4 % d'hydroxyéthylcellulose (HEC) ; de 20 % à 40 % de propylène glycol ; et de 8 % à 40 % de glycérine.

6. Composition pour une utilisation selon la revendication 1, dans laquelle la composition comprend de 0,01 % à 2,5 % de protéine lectine, de 0,005 % à 1,7 % de TRIS, de 0,007 % à 2,4 % de chlorure de sodium (NaCl), de 3 % à 10 % de carboxyméthylcellulose sodique (CMC sodique), de 0,01 % à 4,5 % de poloxamère, de 4 % à 60 % de propylène glycol, de 0,01 % à 4,5 % de saccharose ; et de 0,01 % à 0,2 % de chlorhydrate de L-arginine.

7. Composition pour une utilisation selon la revendication 1, dans laquelle la composition comprend de 0,01 % à 2,5 % de protéine lectine, de 0,005 % à 1,7 % de TRIS ; de 0,007 % à 2,4 % de chlorure de sodium (NaCl) ; de 0,1 à 4 % d'hydroxyéthylcellulose (HEC) ; de 3 % à 10 % de carboxyméthylcellulose sodique (CMC sodique) ; de 4 % à 60 % de propylène glycol ; et de 8 % à 40 % de glycérine.
